# EUROPÄISCHE PATENTSCHRIFT

(11) **EP 1 165 580 B1**
(45) Veröffentlichungstag und Bekanntmachung des Hinweises auf die Patenterteilung: **02.07.2003**
(21) Anmeldenummer: 00925117.4
(22) Anmeldetag: 16.03.2000
(51) Int. Cl.: C07H 15/00

(54) **KATALYTISCHES VERFAHREN ZUR MODIFIZIERUNG VON KOHLENHYDRATEN, ALKOHOLEN, ALDEHYDEN ODER POLYHYDROXYVERBINDUNGEN**
CATALYTIC METHOD FOR MODIFYING CARBOHYDRATES, ALCOHOLS, ALDEHYDES OR POLYHYDROXY COMPOUNDS
PROCEDE CATALYTIQUE PERMETTANT LA MODIFICATION D'HYDRATES DE CARBONE, D'ALCOOLS, D'ALDEHYDES OU DE COMPOSES POLYHYDROXY

(30) Priorität: 16.03.1999 DE 19911504
(43) Veröffentlichungstag der Anmeldung: 02.01.2002
(73) Patentinhaber: Südzucker Aktiengesellschaft, 68165 Mannheim (DE)
(72) Erfinder: CAPAN, Emine, D-38102 Braunschweig (DE); HÄHNLEIN, Marc Sascha, D-68259 Mannheim (DE); PRÜSSE, Ulf, D-38118 Braunschweig (DE); VORLOP, Klaus-Dieter, D-38102 Braunschweig (DE); HAJI BEGLI, Alireza, D-67305 Ramsen (DE)
(74) Vertreter: Schrell, Andreas, Dr.
(86) Internationale Anmeldenummer: EP0002351
(87) Internationale Veröffentlichungsnummer: WO00055165

(56) Entgegenhaltungen:
- EP-A- 0 665 265
- EP-A- 0 857 748
- EP-A- 0 879 642
- WO-A-00/29332
- DE-A- 19 803 891
- PRUESSE, ULF ET AL: "Encapsulation of microscopic catalysts in polymer network gels" CHEM.- ING.-TECH. ( 1997 ), 69(1/2), 100 -103 , 1997, XP000659279 in der Anmeldung erwähnt

## Beschreibung

Die Erfindung betrifft ein Verfahren zur industriellen Umsetzung von Kohlenhydraten, Alkoholen, Aldehyden oder Polyhydroxyverbindungen in wässriger Phase.

In vielen industriellen Prozessen spielt die Umsetzung, z. B. die Oxidation von Kohlenhydraten, Alkoholen, Aldehyden Polyhydroxyverbindungen in wässriger Phase eine entscheidende Rolle und bildet häufig das kritische Stadium von Syntheseverfahren.

So wird beispielsweise die für viele technische Anwendungen benötigte D-Gluconsäure durch eine Oxidation von D-Glucose hergestellt, die als mikrobielle Oxidation mit Aspergillus niger ausgeführt wird.

Eine weitere wichtige Oxidation ist die Bildung von 2-Keto-L-Gulonsäüre aus Sorbose als Zwischenschritt der Herstellung von Ascorbinsäure (Vitamin C). Das klassische Reichstein-Verfahren sieht hierfür eine Zweistufen-Reaktion vor, bei der aufwendig eine L-Sorbofuranose gebildet wird, die anschließend zu der 2-Keto-L-Gulonsäure, beispielsweise auf elektrochemischem Wege oder katalytisch mit Nickeloxid, oxidiert wird.

Die Hydrierung von reduzierenden Mono- und Disacchariden mit Edelmetall-Träger-Katalysatoren wird in der DE 19523008 Al beschrieben. Für die industrielle Produktion, also im großtechnischen auf große Umsätze ausgelegten Maßstab, sind derartige Katalysatoren jedoch nicht geeignet, so dass im industriellen Maßstab im Allgemeinen Raney-Nickel-Kontakte eingesetzt werden.

Bei der reduktiven Aminierung von reduzierenden Zuckern mit Alkylaminen zu Alkylaminopolyolen werden normalerweise Raney-Nickel-Katalysatoren eingesetzt. Ein Nachteil dieser Kontakte ist die sehr kurze Standzeit (Dissertation M. Schüttenhelm, 1995, TU Braunschweig), so dass eine erfolgreiche industrielle Umsetzung aufgrund hoher Katalysatorkosten bisher scheiterte. Weiterhin hat man bei der weiteren Aufarbeitung mit gelösten beziehungsweise komplexierten Ni-Bestandteilen zu rechnen, die die weitere Verwendung des resultierenden Produktes nur durch Einsatz nachgeschalteter aufwendiger und kostenintensiver Reinigungsverfahren erlauben.

Alternativ wurde die Herstellung dieser Produkte mit Pd-Trägerkatalysatoren untersucht. Hier zeigte sich ein Metallverlust, der zum einen die Aktivität des Katalysators erheblich verringerte und aufgrund von Wirtschaftlichkeitsbetrachtungen dessen Einsatz verhindert (Dissertation R. Cartarius, 1999, TU Darmstadt).

Es ist also grundsätzlich z. B. aus EP 0 201 957 A2, WO 97/34861, US 5,643,849 oder Tetrahedron Letters 38 (1997), 9023-9026 bekannt, derartige Reaktionen, insbesondere Oxidationen, katalytisch, insbesondere unter Einsatz von Edelmetallkatalysatoren, vorzunehmen, wobei milde Reaktionsbedingungen hinsichtlich des pH-Wertes und der Reaktionstemperatur ermöglicht werden. Besonders geeignete Katalysatormetalle sind dabei Platin, aber auch Palladium und eventuell Rhodium, wobei grundsätzlich alle Edelmetalle unter Berücksichtigung ihrer Aktivität und ihrer Sauerstofftoleranz in Frage kommen.

Der industrielle Einsatz der theoretisch möglichen katalytischen Oxidation ist bis heute jedoch an Desaktivierungserscheinungen der Katalysatoren gescheitert (vgl. Mallat, Baiker "Oxidation of alcohols with molecular oxygen on platinum metal catalysts aqueous solutions" in Catalysis Today 19 (1994), S. 247 - 248). Die Desaktivierung der Katalysatoren wird dabei auf die Bildung von Katalysatorgiften, eine Überoxidation der Edelmetalloberfläche und auf eine Oberflächenkorrosion und -umstrukturierung des Edelmetalls zurückgeführt. Da einige der Desaktivierungseffekte des Katalysators irreversibel sind und daher durch eine Regenerierung nicht behoben werden können, scheitert die industrielle Anwendung an der geringen Standzeit der Katalysatoren und dem damit erforderlichen hohen Einsatz an Edelmetallmaterial, der das Verfahren unwirtschaftlich macht. Die aufgrund der Desaktivierungseffekte auftretende Metallablösung verursacht nicht nur hohe Kosten für das eingesetzte Edelmetall, sondern führt auch zur Kontamination des katalytisch hergestellten Produktes.

Eine vorgeschlagene Verwendung von mit Promotermetallen versehenen Edelmetallkatalysatoren hat eine gewisse Verringerung der irreversiblen Desaktivierungseffekte erbracht, die jedoch bei weitem noch nicht ausreicht, dass katalytische Oxidationsverfahren wirtschaftlich konkurrenzfähig zu bisher angewendeten Verfahren zu machen.

Die gravierenden Desaktivierungseffekte für die Durchführung einer Oxidationsreaktion haben daher dazu geführt, die Anwendung von Edelmetallkatalysatoren in der Praxis nur noch für bezüglich der Deaktivierung weniger aggressive Reaktionen durchzuführen, insbesondere für Hydrierreaktionen. Bei der Weiterentwicklung der Katalysatoren für diesen Zweck ist die Vergrößerung der Katalysatoroberfläche durch die Bildung von feinen Edelmetallpartikeln durch eine Herstellung des Katalysators aus einem Kolloid durchgeführt worden. Die Separierung der Partikel voneinander und eine Verhinderung eines Zusammenbackens der Partikel ist dadurch erreicht worden, dass das Kolloid mit einem geeigneten Polymer versehen worden ist, so dass die Partikel durch eine Polymerhülle umschlossen sind. Es ist dabei auch angestrebt worden, die Anfälligkeit der Metallpartikeloberflächen gegen Desaktivierung, beispielsweise durch Katalysatorgifte, zu vermindern. Es sind daher für Hydrierreaktionen von kleinen Molekülen, bei denen die Reaktion ohne Diffussionslimitierung verläuft, Metallkatalysatoren eingesetzt worden, die aus polymergeschützten Pt- oder Pd-Partikeln gebildet worden sind.

So offenbart Chem. Ing. Technik 69 (1997), 100-103 Palladium-Trägerkatalysatoren in millimetergroßen gelförmigen Polymernetzwerden zur Reduktion von Nitrit. Nitrit ist ein sehr kleines Molekül, bei dem die Reduktion ohne Diffusionslimitierung verläuft. Hydrierreaktionen mit Metallkatalysatoren, die aus polymergeschützten Pt- oder Pd-Partikeln gebildet wurden, sind für die Umsetzung von relativ großen Molekülen, wie beispielsweise Kohlenhydraten, im Stand der Technik nicht offenbart.

Zur Gewährleistung einer gleichmäßigen Verteilung der Partikel ist auch vorgeschlagen worden, die Partikel mit Tensiden zu umgeben, um beim Aufbringen auf einen Träger eine gleichmäßige Verteilung zu erzielen. Bei dieser Technik wird die Tensidhülle allerdings nach der gleichmäßigen Verteilung der Partikel aufgelöst, um die Katalysatorwirkung zu erzielen, so dass die einzige Funktion des Tensids in der Erzielung der gleichmäßigen Verteilung liegt.

Es ist auch vorgeschlagen worden, polymergeschützte Partikel-Katalysatoren als Bimetall- oder gar Trimetallkatalysatoren auszubilden. Während die Kombination von Edelmetallen der Selektivitätssteuerung dient, lässt die Kombination von Edelmetall mit einem oder zwei Promotormetallen Erfolge bei der Verminderung der Desaktivierung der Katalysatoren zu. Dadurch wurden für die Hydrierreaktion und gegebenenfalls andere Reduktionsreaktionen Perspektiven für eine praktische Anwendung eines katalytischen Verfahrens eröffnet. Die bezüglich der Desaktivierung des Katalysators wesentlich aggressivere Oxidation ist in dieser Hinsicht wegen der bestehenden Aussichtslosigkeit gar nicht mehr untersucht worden.

Für die gattungsgemäßen Reaktionen muss daher weiter auf die bezüglich der Umwelteinflüsse aggressiven oder nur sehr umständlich zu beherrschenden bekannten Verfahren zurückgegriffen werden, obwohl erhebliche Bemühungen unternommen worden sind, zu einfacheren und unter milderen Reaktionsbedingungen ablaufenden Verfahren zu gelangen.

Ausgehend von dem Bestreben, ein industriell anwendbares Verfahren zur Umsetzung, insbesondere Oxidation, Hydrierung oder reduktiven Aminierung von Kohlenhydraten, Alkoholen, Aldehyden oder Polyhydroxyverbindungen in wässriger Phase anzugeben, das unter milderen Reaktionsbedingungen abläuft, wird erfindungsgemäß vorgesehen, dass die Umsetzung katalytisch unter Verwendung eines aus polymerstabilisierten Nanopartikeln gebildeten Metallkatalysators vorgenommen wird.

Die vorliegende Erfindung beruht auf der für die Fachwelt absolut überraschenden und nicht zu erwartenden Erkenntnis, dass aus polymerstabilisierten Nanopartikeln gebildete Metallkatalysatoren bei der katalytischen Umsetzung, insbesondere Oxidation, Hydrierung oder reduktiven Aminierung von Kohlenhydraten, Alkoholen, Aldehyden oder Polyhydroxyverbindungen in wässriger Phase nicht desaktiviert werden, solange die stabilisierende Wechselwirkung zwischen Polymer und Nanopartikeln erhalten bleibt. Dabei ist es erfindungsgemäß nicht erforderlich, dass dem Edelmetallkatalysator ein Promotermetall zugegeben wird, auch wenn dies bei dem erfindungsgemäßen Verfahren naturgemäß selbstverständlich möglich ist. Überraschend für den Fachmann ist weiterhin, dass die bekannten, aus polymergeschützten Partikeln gebildeten Metallkatalysatoren für Hydrierreaktionen für große Moleküle, wie Kohlenhydrate und andere, eingesetzt werden können, bei denen der Fachmann eine Diffussionslimitierung erwartet hätte. Die katalytische Umsetzung von Kohlenhydraten mit diesen Katalysatoren verläuft trotz der die aktiven Zentren umgebenden Polymermatrix überraschend mit hohen Reaktionsgeschwindigkeiten und Selektiven ab. Der Fachmann hätte erwartet, dass im Vergleich zu den bekannten Reaktionen mit Nitrit die großen Kohlenhydratmoleküle, das aktive Zentrum aufgrund der umgebenden Polymermatrix beziehungsweise einer Diffusionslimitierung nur begrenzt für eine Reaktion zur Verfügung stehen. Es konnte jedoch gezeigt werden, dass sogar die großen Di- und Oligosaccharidmoleküle mit dem erfindungsgemäßen Katalysatorsystem vorteilhaft umgesetzt werden können.

Die vorliegende Erfindung betrifft insbesondere Verfahren zur industriellen Umsetzung von Edukten, ausgewählt aus der Gruppe bestehend aus Alkoholen, Aldehyden, und/oder Polyhydroxyverbindungen wie Kohlenhydrate, Kohlenhydratderivate, Stärkehydrolysate, insbesondere Mono-, Di- oder Trisaccharide, in wässriger Phase, wobei die Umsetzung katalytisch unter Verwendung eines aus polymerstabilisierten Nanopartikeln gebildeten Metallkatalysators vorgenommen wird. Es kann vorgesehen sein, auch Gemische der genannten Edukte gemeinsam umzusetzen.

In einer bevorzugten Ausführungsform der vorliegenden Erfindung ist die Umsetzung eine Oxidation der genannten Edukte, wobei vorzugsweise Kohlenhydrate zum Beispiel Glucose, Sorbose, Saccharose, Maltose, Lactose, Stärkehydrolysate und/oder Isomaltulose zu den entsprechenden Kohlenhydratsäuren oxidiert werden. Aufgrund der recht aggressiven Bedingungen bei Oxidationen ist in dieser Ausführungsform die erfindungsgemäß beobachtete Langzeitstabilität und das nicht auftretende Metall-Leaching besonders überraschend.

In einer weiteren Ausführungsform ist die Umsetzung eine Reduktion, insbesondere eine Hydrierung, wobei reduzierende Zucker, wie zum Beispiel Glucose, Fructose, Xylose, Sorbose, Isomaltose, Isomaltulose, Trehalulose, Maltose und/oder Lactose zu den entsprechenden Zuckeralkoholen hydriert werden. Auf diese Weise können zum Beispiel aus Isomaltulose, Isomalt, 1.1-GPM (1-0-α-D-Glucopyranosyl-D-mannit)-oder 1.6-GPS (6-0-α-D-Glucopyranosyl-D-sorbit) angereicherte Gemische gewonnen werden. Derartige angereicherte Gemische werden in der DE 195 31 396 C2 beschrieben.

In einer weiteren Ausführungsform kann die industrielle Umsetzung der genannten Edukte eine reduktive Aminierung sein, wobei vorzugsweise reduzierende Zucker reduktiv aminiert werden, insbesondere Glucose, Fructose, Xylose, Sorbose, Isomaltose, Isomaltulose, Trehalulose, Maltose und/oder Lactose.

In bevorzugter Ausführungsform ist der Metallkatalysator ein Katalysator, der im Wesentlichen aus Edelmetall besteht oder dieses enthält, wobei das Edelmetall zum Beispiel Platin, Palladium, Rhodium und/oder Ruthenium sein kann. Der Metallkatalysator kann aber auch ein Katalysator sein, der im Wesentlichen aus einem Nicht-Edelmetall besteht oder dieses enthält, wobei das Nicht-Edelmetall zum Beispiel Kupfer und/oder Nickel sein kann.

Im Zusammenhang mit der vorliegenden Erfindung findet die Umsetzung in wässriger Phase statt, wobei die Umsetzung vorzugsweise bei einer Temperatur von 35 bis 120°C und einem pH-Wert von 5 bis 12 stattfindet.

Im Zusammenhang mit der vorliegenden Erfindung wird unter einem polymerstabilisierten Nanopartikel ein Metallpartikel verstanden, um den eine Polymerhülle gebildet ist, wobei der Gesamtdurchmesser des polymerumhüllten Metallpartikels, als Metallpartikelkern plus Hülle vorzugsweise in einem Bereich von 3 bis 200 Nanometer liegt.

Die Erfindung sieht in besonders bevorzugter Weise vor, dass die umzusetzenden Alkohole, Aldehyde oder Polyhydroxyverbindungen, insbesondere Kohlenhydrate, Kohlenhydratderivate oder ähnliches in wässriger Lösung umgesetzt werden, wobei Konzentration von 0,1 bis 60 % bevorzugt werden. So kann die Glucose zum Beispiel in Form von Glucosesirup vorliegen.

Insbesondere kann in einer weiteren bevorzugten Ausführungsform vorgesehen sein, die erfindungsgemäß umgesetzten vorstehend genannten Produkte bei der Oxidation nach ihrer Umsetzung zu einer Produktlösung einer Elektrodialyse zuzuführen und so die Produkte aus der erhaltenen Produktlösung zu entfernen und zu gewinnen. Eine derartige besonders bevorzugte Verfahrensweise eignet sich zum Beispiel zur Herstellung von monooxidierten Kohlenhydraten beziehungsweise Kohlenhydratderivaten und primären Alkoholen. Die Abtrennung der Oxidationsprodukte mittels Elektrodialyse, zum Beispiel wie in der EP 0 651 734 B1 beschrieben, führt zum Erhalt von praktisch reinen Produkten.

Das erfindungsgemäße Verfahren kann in bevorzugter Weise also mit einem Verfahren und der dazugehörigen Vorrichtung gemäß EP 0 651 734 B1 gekoppelt werden, um die gewünschten Produkte mittels Elektrodialyse besonders rein zu erhalten. Die Lehre aus EP 0 651 734 B1 ist vollständig in den Offenbarungsgehalt der vorliegenden Lehre hinsichtlich der dort beschriebenen elektrodialytischen Abtrennung aufgenommen, wobei dafür auch Schutz begehrt wird.

Für einen ständig wiederholten Einsatz der erfindungsgemäßen Katalysatorpartikel ist in Betracht zu ziehen, dass die Polymerhülle um die Nanopartikel abgelöst bzw. verbraucht wird. Es ist daher erfindungsgemäß besonders bevorzugt, wenn in die wässrige Phase kontinuierlich oder in geeigneten Zeitabständen eine Zugabe des die Nanopartikel stabilisierenden Polymers erfolgt, um auf diese Weise sicherzustellen, dass die wirksame Polymerhülle um die Nanopartikel erhalten bleibt.

Bei dem erfindungsgemäßen Verfahren können die Nanopartikel in an sich bekannter Weise auf einem Trägermaterial immobilisiert, also geträgert werden, wobei als Trägermaterial vorzugsweise ein poröses Material in zusammenhängender Form oder in Pulverform verwendet wird oder die polymer stabilisierten Nanopartikel in einer Gelstruktur immobilisiert werden.

Als Immobilisisierungsmaterialien mit Hilfe einer Adsorption kommen insbesondere in Frage: Al₂O₃, SiO₂, TiO₂, ZrO₂, Aktivkohle, Polymerlatex, Polystyrollatex, Polyacrylamid-Gel, Deloxan (Alkylsulfonsäurepolysiloxan), Aminoethyl Bio-Gel P-150. Eine Einschlußimmobilisierung kann in bevorzugter Ausführungsform in Alginaten, Polyvinylalkohol, Polyurethanen oder ähnliches erfolgen.

Sofern in einer Ausführungsform der Erfindung wie vorstehend beschriebene immobilisierte Trägerkatalysatoren eingesetzt werden, können die erfindungsgemäßen polymerstabilisierten und/oder geträgerten Nanopartikel bevorzugt in Gelen, besonders Hydrogelen, homogen oder inhomogen verteilt oder auch an der Oberfläche lokalisiert sein. Neben den Trägermaterialien Aluminiumoxid, Siliciumdioxid und/oder Titandioxid kommen dafür auch Aktivkohle, Alumosilikate und Ionentauscherharze oder ähnliches in Betracht.

Schließlich sind in einer weiteren Ausführungsform auch Membrananordnungen möglich, bei denen die aktive Komponente, das heißt die polymerstabilisierten Nanopartikel, gegebenenfalls auch in geträgerter Form, auf oder zwischen Membranen (zum Beispiel Hohlfasern, Diffusionsmembranen, poröse Membranen und Flachmembranen) aufgebracht sind.

Als die Nanopartikel schützende und umhüllende Polymere kommen in bevorzugter Ausführungform zahlreiche Homopolymere, Copolymere und insbesondere Block-Copolymere und Pfropf-Copolymere in Frage. Insbesondere sind zu nennen Polyvinylpyrrolidone und geeignete Derivate, Polyvinylalkohol, Polyacrylsäure, Poly(2-Ethyl-2-Oxazolin), Poly(2-Hydroxypropylmethacrylat), Poly(Methylvinylether-Co-Maleinanhydrid), Polymethacrylsäure, Poly(1-Vinylpyrrolidon-Co-Acrylsäure), Poly(Styrolsulfon-säure) Poly(2-Acrylamido-2-Methyl-1-propansulfonsäure), Poly(Vinylphosphon-säure), Polydiallyldimethylammoniumchlorid (PDADMAC), Polymethacrylamidopropyltrimethylammoniumchlorid,Poly(3-Chloro-Hydroxy-Propyl-2-Methacryloxy-Ethyldemethylammoniumchlorid).

Die erfindungsgemäßen Katalysatoren können in bevorzugter Ausführungsform auch als Kolloide/Cluster verwendet werden, wobei die aktive Komponente in Form freier, das heißt nicht immobilisierter Kolloide oder Cluster vorliegt. Die größte Anordnung dieser Kolloide/Cluster liegt erfindungsgemäß im Nanometerbereich, das heißt in einem Bereich von 1 nm bis 20 nm. Wesentlich ist lediglich, dass die Kolloiedteilchen und Cluster durch eine schützende Polymerhülle umgeben sind.

Die Katalysatoren können entsprechend der Art des Katalysators und des jeweiligen Reaktors zum Beispiel als Kugeln, Perlen, Zylinder, Hohlzylinder, Netze, Pulver, Presslinge, Granulate, Hohlkugeln, Fasern und Folien ausgeführt sein. Das Verfahren selbst kann sowohl in kontinuierlich, semikontinuierlich oder auch in absatzweise arbeitenden Anlagen angewendet werden. Als Reaktoren kommen je nach verwendetem Katalysator zum Beispiel Festbettreaktoren, Reaktoren mit expandierenden Festbetten, Fließbettreaktoren, Wirbelschichtreaktoren, Rührschichtreaktoren, Rührreaktoren und Membranreaktoren in Betracht. Diese Systeme können mit oder ohne Katalysator- bzw. Flüssigkeitsrückführung betrieben werden. Die Systeme können soweit erforderlich auch mit geeigneten Bauteilen zur Katalysatorrückhaltung, zum Beispiel mit Zyklonen, Filtern und Membranen versehen werden.

Weitere vorteilhafte Ausgestaltungen ergeben sich aus den Unteransprüchen.

Die Erfindung werden anhand der folgenden Beispiele und der dazugehörigen Figuren näher erläutert.

Die Figuren erzeigen
**Figur 1:** Messergebnisse zur Sorboseoxidation mittels eines erfindungsgemäß eingesetzten Katalysators,
**Figur 2:** Messergebnisse zur Sorboseoxidation mittels eines Vergleichskatalysators,
**Figur 3:** Messergebnisse zur Sorboseoxidation mittels eines erfindungsgemäß eingesetzten Katalysators,
**Figur 4:** Messergebnisse zur Glucoseoxidation mittels eines erfindungsgemäß eingesetzten Katalysators,
**Figur 5:** Messergebnisse zur Oxidation von Saccharose mittels eines erfindungsgemäß eingesetzten und eines Vergleichskatalysators,
**Figur 6:** Messergebnisse zur reduktiven Aminierung von Isomaltulose mittels eines erfindungsgemäß eingesetzten und eines Vergleichskatalysators,
**Figur 7:** Messergebnisse zur Hydrierung von Isomaltulose mittels eines erfindungsgemäß eingesetzten und eines Vergleichskatalysators.

### Beispiel 1:

### Herstellung von PVP-stabilisierten Platinkolloiden

3,27 g Polymer, nämlich Polyvinylpyrrolidon-PVP, werden in 33 ml Methanol gelöst, gegebenenfalls muss die Lösung leicht erhitzt werden, damit sich das Polymer vollständig löst. Nach dem Auflösen des Polymers werden 398,2 mg (0,769 mmol) Platin(IV)-chlorowasserstoffsäure-hexahydrat (H₂PtCl₆·6H₂O) (Platingehalt 150 mg) und 291,6 mg (7,29 mmol) NaOH dazugegeben und unter Rückfluss gekocht. Auch hierbei verfärbt sich die Lösung bei der Zugabe von Platin (IV)-chlorowasserstoffsäure-hexahydrat gelb. Nach der Reduktion wird weitere 60 Minuten unter Rückfluss gekocht. Die Reduktion erfolgt erst nach ca. 30 minütigem Sieden schlagartig. Die Reduktion ist auch hierbei daran zu erkennen, dass sich ein braun-schwarzes kolloidales Sol bildet. Nach dem Erkalten des Sols wird der nicht umgesetzte Alkohol dialytisch entfernt. Bei der Dialyse wird das kolloidale Sol kontinuierlich durch das intrakappilare Volumen eines Hohlfaserdialysatormoduls (Fresenius Modell F5 HPS) im Gegenstrom zu deionisiertem Wasser im extrakapillaren Volumen umgepumpt. Beim Dialysieren wird das kolloidale Sol vollständig zurückgehalten.

### Beispiel 2:

### Trägerung des Pt-Kolloids auf Al₂O₃

4,69 g Al₂O₃ (HL 1000) in Form von hochporösen Partikeln werden zu einer 50 mg Pt enthaltenden kolloidalen Lösung gegeben. Anschließend werden 1,15 ml Ameisensäure dazugegeben und über Nacht gerührt. Die Lösung wird im Laufe der Zeit klar. Das Reaktionsgemisch wird über eine G4 Fritte abgefrittet. Der Feststoff wird erst mit Methanol und anschließend mit destilliertem Wasser gewaschen und im Trockenschrank getrocknet.

### Beispiel 3:

### Sorboseoxidation

Zur Bestimmung der Sorboseabbauaktivität wird der Reaktor mit 150 ml Katalysatorsuspension befüllt. Vor den Einsätzen wird die Reaktionssuspension 30 Minuten mit Wasserstoff begast, um andere Gase, vor allem Sauerstoff, aus der Reaktionslösung zu verdrängen und um den Katalysator zu aktivieren. Um den gelösten Wasserstoff aus der Reaktionssuspension zu strippen, wird die Lösung ca. 15 Minuten mit Stickstoff begast. Unter Stickstoffbegasung werden 7,5 g Sorbose zu der Lösung gegeben und auf 50 ° C Reaktionstemperatur erwärmt. Anschließend wird der pH-Wert auf pH 7,3 eingestellt. Nachdem die Reaktionssuspension 50°C erreicht hat, wird sie dann mit Sauerstoff begast, d. h. die Reaktion ist damit gestartet, wobei die Begasungsrate am Anfang sehr hoch ist, damit man eine schnelle Sättigung der Reaktionssupension erreicht. Nachdem man eine vollständige Sättigung (ca. 95 % )erreicht hat, wird die Begasungsrate reduziert. Der Sättigungsgrad wird mittels der Sauerstoffelektrode überwacht und die Begasungsrate gegebenenfalls wieder erhöht, so dass die Reaktion über den gesamten Reaktionsverlauf gesättigt bleibt. Die Reaktionsdauer betrug pro Einsatz 24 Stunden. Danach wurde der Katalysator der erwähnten Regenerationsbehandlung unterworfen und ist dann für den nächsten Einsatz vorbereitet.

### Beispiel 4:

### Vergleichsversuch

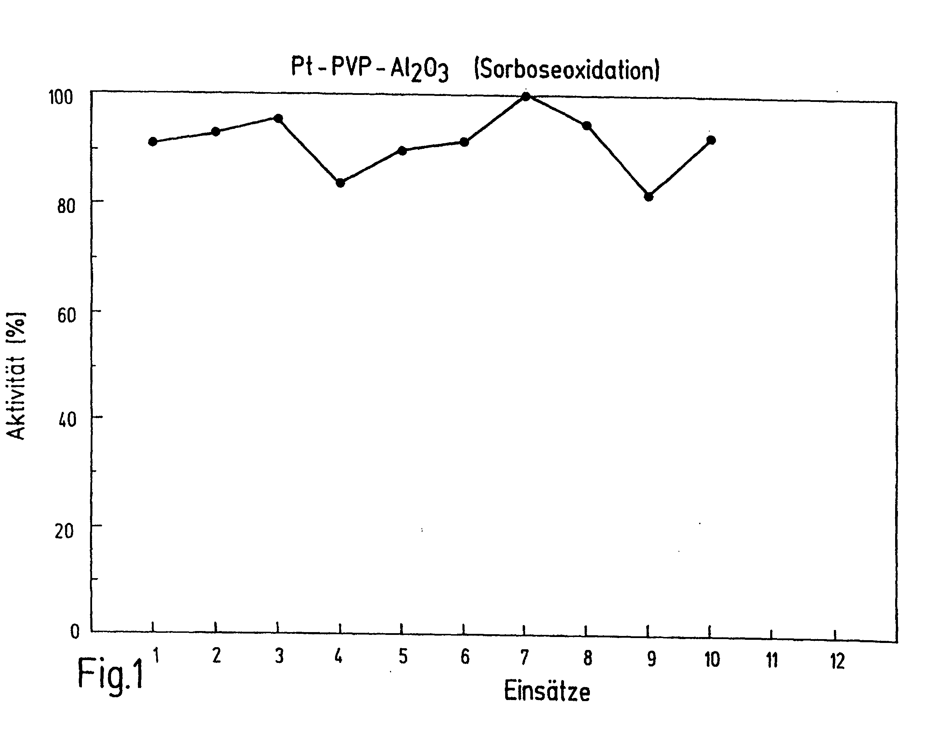

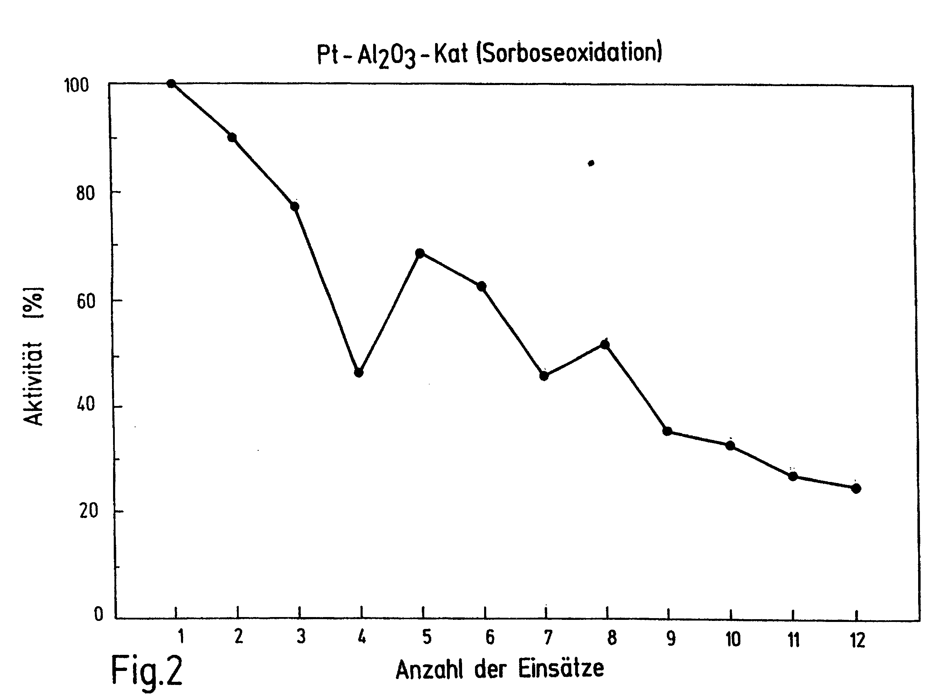

Für die in Beispiel 3 dargestellte Sorboseoxidation ist der gemäß Beispiel 1 und 2 hergestellte polymergeschützte Platinkolloidkatalysator in wiederholten Reaktionsläufen auf seine Aktivität hin untersucht worden. Die dabei entstandenen Messergebnisse sind in **Figur 1** dargestellt und zeigen, dass die Aktivität des Katalysators über viele Einsätze (von je 24 Stunden) praktisch unverändert bleibt, während ein herkömmlicher Platinkatalysator auf Al₂O₃ nach vergleichbaren Einsätzen eine auf 20 bis 30 % reduzierte Aktivität aufweist, wie **Figur 2** verdeutlicht. Mittels Atomabsorptionsspektroskopie wurde ermittelt, dass der herkömmliche Platinkatalysator nach 6 Einsätzen bereits 28 % Platin verloren hatte, während der erfindungsgemäße Katalysator keine Verluste aufwies.

### Beispiel 5:

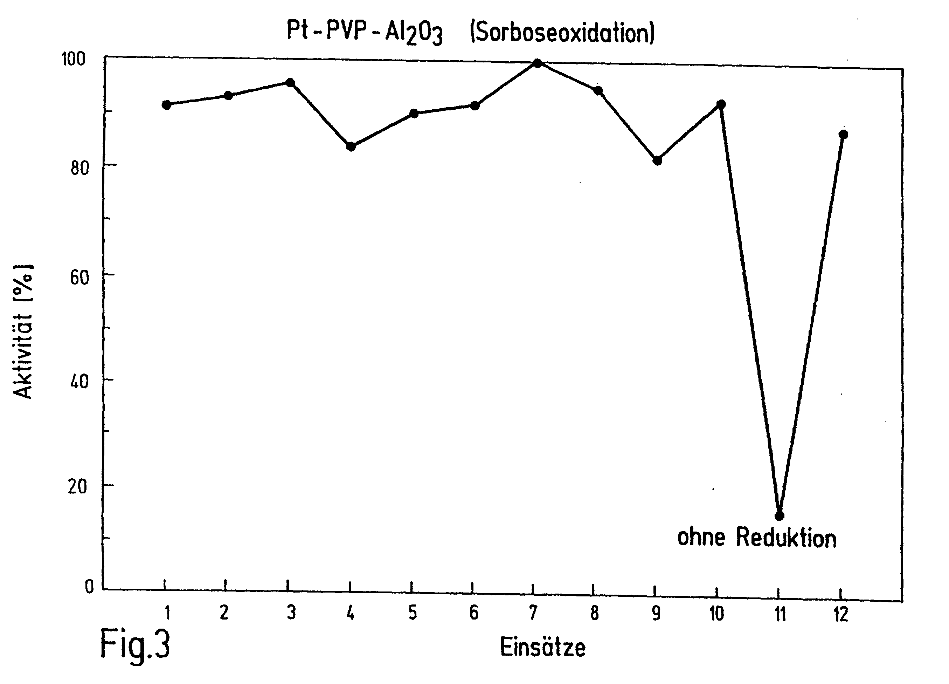

**Figur 3** verdeutlicht den Aktivitätsverlauf bei einem erfindungsgemäßen Katalysator analog **Figur 1**, wobei die ansonsten durchgeführte regenerierende Begasung mit Wasserstoff gemäß Beispiel 3 unterlassen worden ist, wodurch die Aktivität beim elften Einsatz erheblich reduziert worden ist. Durch eine danach erfolgende Begasung mit Wasserstoff wird jedoch die ursprüngliche Aktivität wieder hergestellt, wie sich am zwölften Einsatz gemäß **Figur 3** zeigt. Die Desaktivierung ohne Regenerationsbehandlung ist daher reversibel.

### Beispiel 6:

### Glucoseoxidation

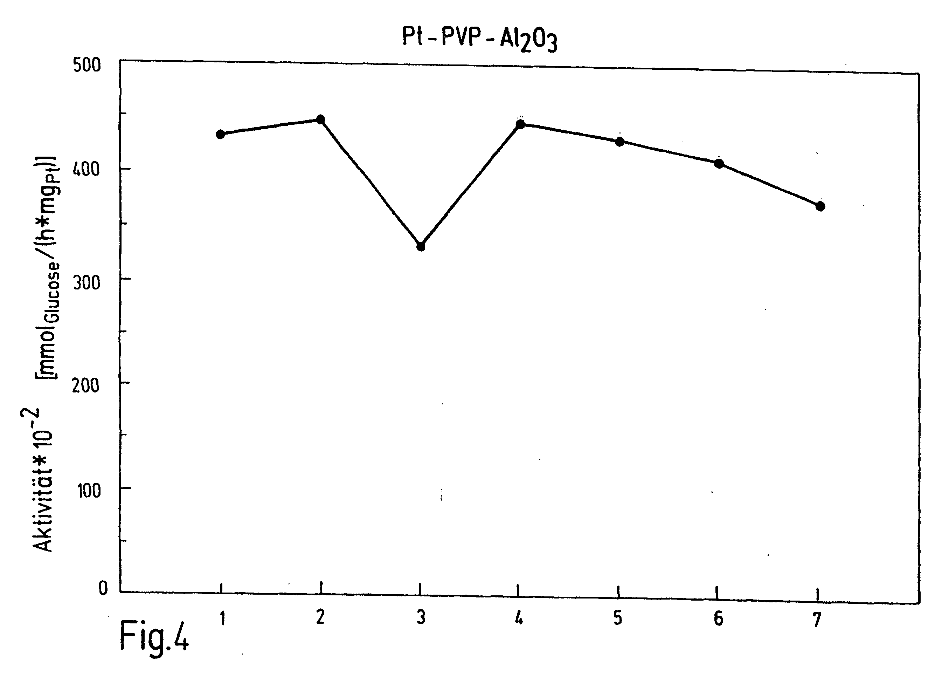

Zur Bestimmung der Glucoseabbauaktivität wird der Reaktor mit 100 ml Katalysatorsuspension befüllt. Vor den Einsätzen wird die Reaktionssuspension 15 Minuten mit Stickstoff begast, um andere Gase, vor allem Sauerstoff, aus der Reaktionslösung zu verdrängen. Unter Stickstoffbegasung werden 10 g Glucose zu der Lösung gegeben und auf 50°C Reaktionstemperatur erwärmt. Anschließend wird der pH-Wert auf pH 9,5 eingestellt. Nachdem die Reaktionssuspension 50°C erreicht hat, wird sie dann mit Sauerstoff begast, d. h. die Reaktion ist damit gestartet, wobei die Begasungsrate am Anfang sehr hoch ist, damit man eine schnelle Sättigung der Reaktionssuspension erreicht. Nachdem man eine vollständige Sättigung (ca. 95 %) erreicht hat, wird die Begasungsrate reduziert. Der Sättigungsgrad wird mittels der Sauerstoffelektrode überwacht und die Begasungsrate gegebenenfalls wieder erhöht, so dass die Reaktion über den gesamten Reaktionsverlauf gesättigt bleibt. **Figur 4** verdeutlicht, dass die Aktivität des Katalysators nach mehreren Einsätzen zu jeweils 4 Stunden allenfalls schwach zurückgeht, während herkömmliche Katalysatoren nach spätestens 4 Einsätzen praktisch unbrauchbar sind, weil die Aktivität auf 20 % oder darunter abgesunken ist.

### Beispiel 7:

### Herstellung Polymer-stabilisierter Metallkolloide

**Tabelle 1:**

| Qualitative Zusammensetzung der Katalysatoren 1 bis 5 | | | | |
|---|---|---|---|---|
| | **Aktives** **Metall** | **Träger** | **Stabilisierendes** **Polymer** | **Umsetzungstyp** |
| Katalysator 1 | Pt | Al₂O₃ | Polyvinylpyrrolidon | Oxidation |
| Katalysator 2 | Pd | TiO₂ | Poly(1-Vinylpyrrolidon) Co-Acrylsäure | Reduktive Aminierung |
| Katalysator 3 | Ru | Al₂O₃ | Polyvinylpyrrolidon | Hydrierung |
| Katalysator 4 | Cu | Aktivkohle | Polyvinylpyrrolidon | Hydrierung |
| Katalysator 5 | Ni | TiO₂ | Polymethacrylamidopropyltrimethylammoniumchlorid | Hydrierung |

Die Herstellung der Polymer-stabilisierten Metallkolloide erfolgte analog dem Beispiel 1. Die Zusammensetzung der Katalysatoren geht aus Tabelle 1 hervor. Die Einsatzmenge des jeweils eingesetzten Polymers wurde hierbei zunächst konstant gehalten. Die Menge der verwendeten Edelmetallsäuren beziehungsweise Metallsalze wurde so gewählt, dass nach theoretischer vollständiger Umsetzung ein katalytisch aktiver Metallgehalt von 150 mg angenommen werden kann. In der Tabelle 2 werden die Rohstoffe und Mengen für die Herstellung der Katalysatoren aufgezeigt.

**Tabelle 2:**

| Quantitative Zusammensetzung der Katalysatoren 1 bis 5 | | | | |
|---|---|---|---|---|
| | **Aktives** **Metall** | **Ausgangskomponente** | **Menge A.-** **komponente** **in mg** | **Menge in** **mmol** |
| Katalysator 1* | Pt | H₂PtCl₆x 6 H₂O | 398,2 | 0,769 |
| Katalysator 2 | Pd | PdCl₂ | 249,9 | 1,41 |
| Katalysator 3 | Ru | RuCl₃ | 307,9 | 1,48 |
| Katalysator 4 | Cu | CuCl₂ | 319,7 | 2,36 |
| Katalysator 5 | Ni | Ni(NO₃)₂ | 466,9 | 2,56 |

| | | | | |
|---|---|---|---|---|
| * wie bei der Sorboseoxidation in Beispiel 3 verwendet | | | | |

Die Trägerung der Katalysatoren erfolgte analog der in Beispiel 2 aufgeführten Vorgehensweise.

### Beispiel 8:

### Oxidation von Saccharose

Die Oxidation der Saccarose mit dem *Katalysator 1* erfolgt entsprechend dem Beispiel 3.

Die Reaktionstemperatur liegt hier bei 40°C und zur kontinuierlichen Abtrennung der Oxidationssprodukte wird eine Elektrodialyse-Anlage verwendet (ausführlich in Dissertation M. Schüttenhelm, TU Braunschweig und in der EP 0 651 734 B1 beschrieben). Die Anlage wurde 10 Tage betrieben und lieferte hierbei das folgende Produktspektrum:
1-O-(β-D-Fructosylfuranuronyl)-α-D-glucopyranosid: 36 ± 3%
2-O-(α-D-Glucopyranosyl)-β-D-glucofuranonsäure: 37 ± 3%
1-O-(β-D-Fructosylfuranosyl)-α-D-glucopyranuronid: 10 ± 2%
Sonstige, nicht näher charakterisierte Produkte: 5 ± 2 %

Die Aktivität des Katalysators war nahezu über 10 Tage konstant.

Als Vergleich wurde ein nicht polymerstabilisierter kommerziell erhältlicher Kontakt mit Pt auf Aktivkohleträger und einem Metallanteil von 1 Gew.-% getestet, der folgendes Produktspektrum lieferte:
1-O-(β-D-Fructosylfuranuronyl)-α-D-glucopyranosid: 37 ± 3%
2-O-(α-D-Glucopyranosyl)-β-D-glucofuranonsäure: 36 ± 3%
1-O-(β-D-Fructosylfuranosyl)-α-D-glucopyranuronid: 10 ± 2 %
Sonstige, nicht näher charakterisierte Produkte: 13 ± 2%

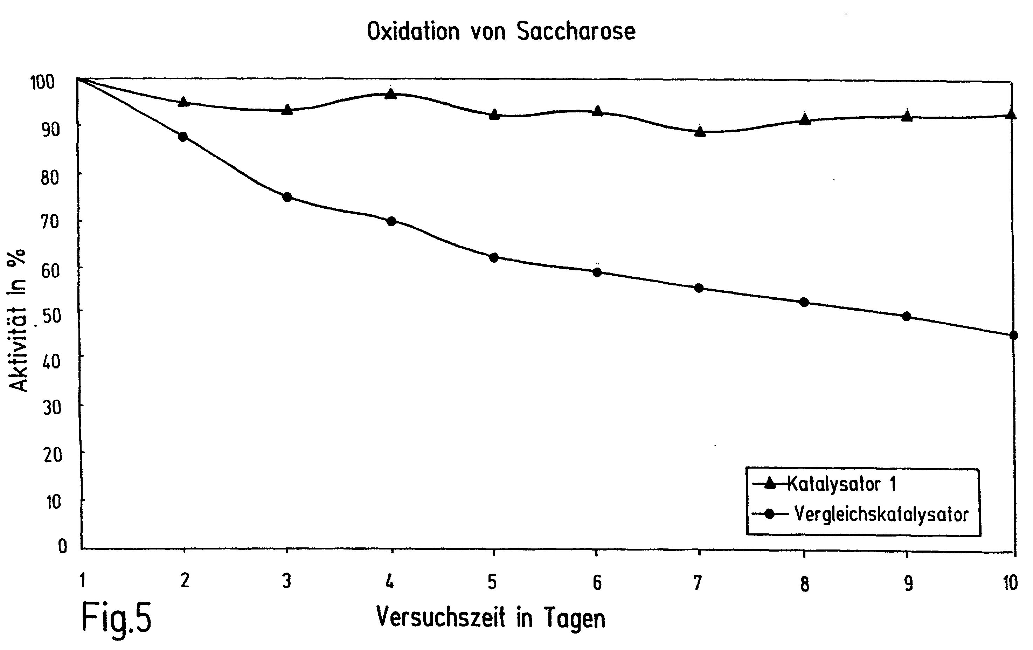

Dieser Vergleichskatalysator lieferte neben den gewünschten Monosaccharosecarbonsäuren einen erheblich höheren Anteil an Nebenprodukten, wie **Figur 5** zeigt. Bereits nach dem dritten Tag konnte eine kontinuierliche Abnahme der Katalysatoraktivität beobachtet werden.

### Beispiel 9:

### Reduktive Aminierung von Isomaltulose (Palatinose) im Suspensionsverfahren

Die Untersuchungen zur reduktiven Aminierung wurden in einem Hochdruckautoklaven im Slurryverfahren unter Verwendung des *Katalysators 2* (vgl. Bsp. 7). (5g) durchgeführt.

Die katalytischen Hydrierungen wurden in einem Labor-Hochdruckautoklaven mit den folgenden Betriebsdaten durchgeführt:

### Autoklav:

- 750 mL-Hochdruckautoklav, thermostatisierbar; max. Arbeitsdruck: 15 MPa (Fa. BERGHOF, Eningen)
- drehzahlgesteuerter, induktiv betriebener Rührer
- innere Temperaturmessung durch PT 100-Widerstandsthermometer
- manuelles Probenentnahme-Nadelventil

### Thermostat:

Kompakt-Kälte-Thermostat RKS 20 D mit externer Regeleinheit (Fa. LAUDA, Lauda-Königshofen)

### Wasserstoffzufuhr:

Flaschenentnahme über Reduzierventile:
<10 MPa: Spüleinheit; 15 MPa: Reaktionsanschluss

### Aminierung mit n-Dodecylamin:

50 g (0,139 mol) Palatinose-Monohydrat (Mᵣ[C₁₂H₂₂O₁₁ · H₂O] = 360,31 g/mol) wurden in einem thermostatisierbaren 55 mL-Doppelwandkolben in einem Gemisch aus 180 mL Wasser und 55 mL 2-Propanol gelöst und auf 10°C gekühlt. Dazu wurde eine Lösung von 7,36 g (0,040 mol) n-Dodexylamin (Mᵣ[C₁₂H₂₇N] = 185,35 g/mol) in 120 mL Wasser und 70 mL 2-Propanol langsam getropft und 1 h gut gerührt. Die so erhaltene Osylaminreaktionslösung wurde in den temperierten Autoklaven überführt, mit dem Katalysator versetzt, danach zügig dreimal mit Wasserstoff gespült und 24 h bei 50 bar und 70°C hydriert. Nach dem Abkühlen auf Raumtemperatur wurde vom Katalysator filtriert und die Rohproduktlösung am Rotationsverdampfer bei 38°C im Wasserstrahlvakuum vorsichtig eingeengt. Der Rückstand wurde anschließend aufgereinigt.

Es zeigte sich, dass der Wasserstoffpartialdruck bei der Hydrierung in bevorzugter Ausführungsform mindestens bei 30 bar liegen sollte, um unerwünschte Nebenreaktionen zu unterdrücken. Selbstverständlich kann auch bei 180 bar oder darüber hydriert werden. Die Versuche erfolgten durchgängig bei 50 bar und einer Temperatur von 70°C.

Die Anlage wurde diskontinuierlich 10 Tage betrieben und alle 24 Stunden mit neuer Eduktlösung befüllt. Der Katalysator wurde während dieser Zeit nicht gewechselt. Als Vergleich wurde ein nicht polymerstabilisierter Kontakt (1% Pd auf TiO₂) geprüft. Die Beurteilung der Aktivität erfolgte durch Ermittlung des Isomaltulose-Umsatzes jeweils nach 24 Stunden. Am Anfang war der Umsatz bei beiden Kontakten nahezu quantitativ (red. Substanzen <0,1 %, also unterhalb der Nachweisgrenze); dieser Wert wurde mit 100 % als Bezugsgröße für die Auswertung der Versuchsreihe gewählt.

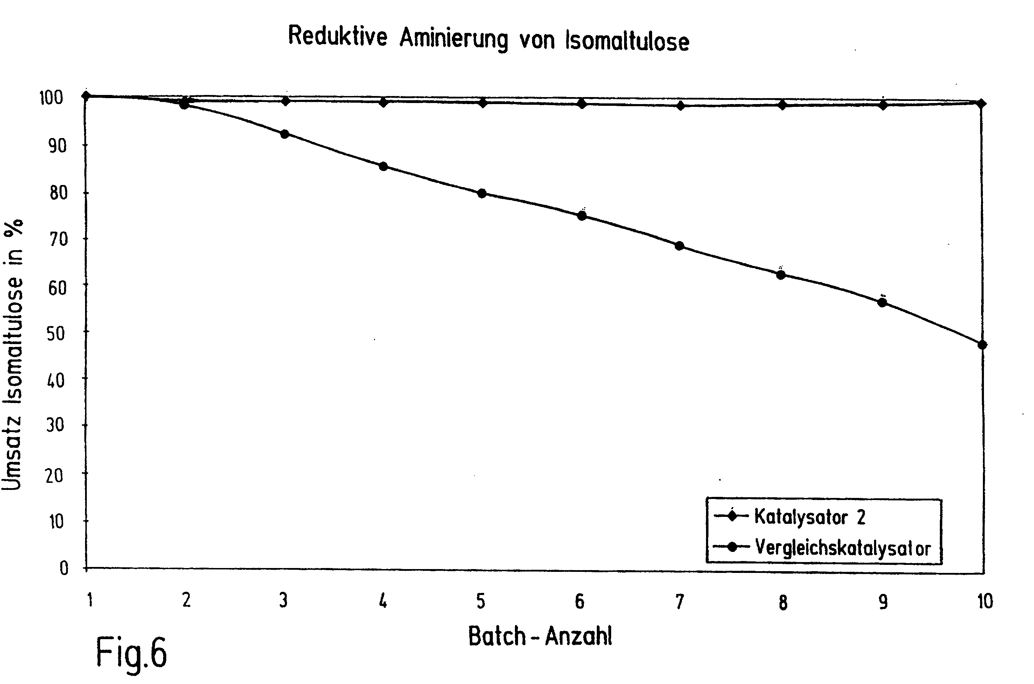

Die Ergebnisse sind in der **Figur 6** dargestellt.

Der nicht polymerstabilisierte Träger verliert bei dieser Reaktion bereits nach dem dritten Tag 15 % seiner Reaktivität; die Reaktivität des polymerstabilisierten Kontaktes verändert sich im untersuchten Zeitraum praktisch nicht.

### Beispiel 10:

### Versuche zur Hydrierung

Im Rahmen der Untersuchungen wurde die Eignung von polymerstabilisierten Kontakten für Hydrierreaktionen untersucht.

Es wurden jeweils 5 g der *Katalysatoren 3 -5* hergestellt und im oben beschriebenen Autoklavensystem mit dem Zucker Isomaltulose getestet. Hierzu wurden jeweils 500 ml Isomaltulose-Lösung mit einem Trockensubstanzgehalt von 30 % in den Autoklaven eingefüllt und 5 g des Katalysators zugesetzt. Als Vergleichskatalysator wurde ein Standardkontakt auf Ni/SiO₂-Basis verwendet. Der Autoklav wurde verschlossen und zur Entfernung des Sauerstoffes wurde dreimal mit Stickstoff gespült. Die anschließenden 10 Batch-Hydrierungen für jeden Katalysator wurden bei folgender Parametereinstellung durchgeführt:

| | |
|---|---|
| Reaktionstemperatur | 70°C |
| Wasserstoffpartialdruck | 150 bar |
| Rührerdrehzahl | 700 UpM |
| Reaktionszeit | 24 h |

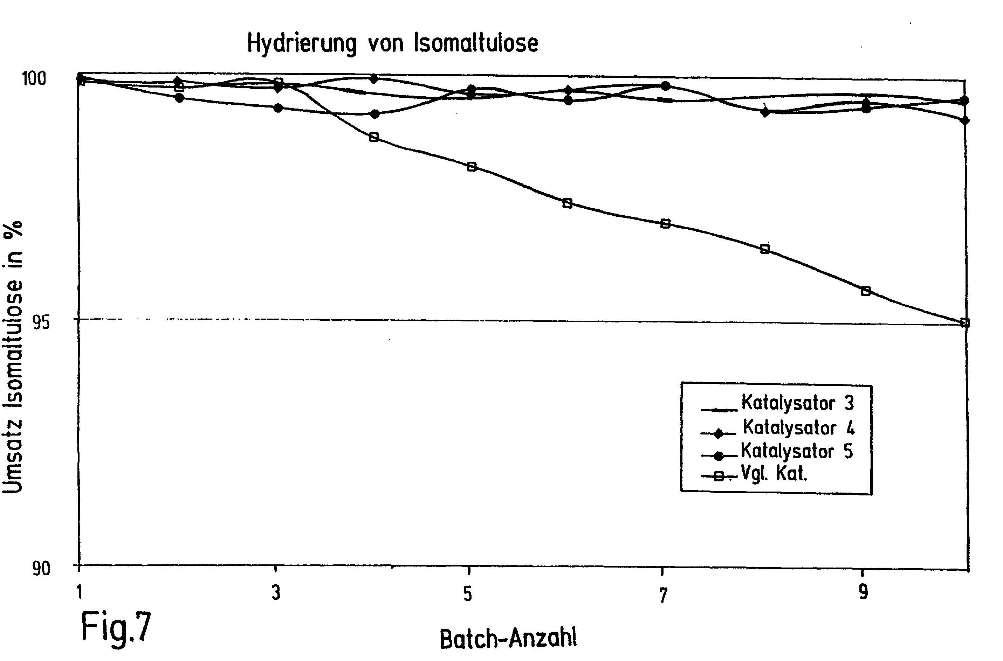

Die Hydrierung der Isomaltulose liefert als Hauptprodukte ein Polyol-Isomeren-Gemisch bestehend aus 6-O-α-D-Glucopyranosyl-D-sorbit (1,6-GPS) und 1-0-α-D-Glucopyranosyl-D-mannit (1,1-GPM). Die Beurteilung der Aktivität erfolgte durch Ermittlung des Isomaltuloseumsatzes nach 24 Stunden. Die **Figur 7** zeigt, dass bei den Katalysatoren 3 bis 5 im Untersuchungszeitraum keine Abnahme der Reaktivität zu beobachten ist, während bei dem Vergleichskatalysator bereits ab der 5. Hydrierung eine Abnahme der Reaktivität beobachtet wird.

Je nach verwendetem Metall und Träger der verwendeten Katalysatoren können die Mengen-Verhältnisse bezüglich des 1,6-GPS- und 1,1-GPM-Anteils der Produktlösungen selektiv gesteuert werden. Durch die Wahl des Katalysators kann - wie aus **Tabelle 3** ersichtlich - die Selektivität der Hydrierreaktion also so beeinflusst werden, dass eine gezielte Herstellung einer entsprechend 1,6-GPS und 1,1-GPMangereicherten Produktlösung möglich ist.

**Tabelle 3:**

| Die Selektivität der Hydrierreaktion | |
|---|---|
| **Katalysator** | **Selektivität** |
| *Katalysator 3* | 1,6-GPS-selektiv |
| *Katalysator 4* | 1,1-GPM-selektiv |
| *Katalysator 5* | äquimolares Verhältnis |
| Vergleichskatalysator | äquimolares Verhältnis |

Die aufgeführten Beispiele belegen, dass trotz unterschiedlicher Kombinationen von unterschiedlichen Metallen, Polymeren und Trägern eine Vielzahl von im Prinzip gleichen Katalysatoren herstellbar ist, denen gemeinsam ist, dass sie insbesondere im wässrigen Medium eine bedeutend höhere Stabilität hinsichtlich Haftung und Beladung der aktiven Metallkomponente und somit längere Standzeiten aufweisen, als herkömmlich verwendete Katalysatoren.

## Patentansprüche

1. Verfahren zur industriellen Umsetzung von Kohlenhydraten, Alkoholen, Aldehyden oder Polyhydroxyverbindungen in wässriger Phase, **dadurch gekennzeichnet, dass** die Umsetzung katalytisch unter Verwendung eines aus polymerstabilisierten Nanopartikeln gebildeten Metallkatalysators vorgenommen wird.

2. Verfahren nach Anspruch 1, wobei die Umsetzung eine Oxidation ist.

3. Verfahren nach Anspruch 2, wobei Glucose, Fructose, Sorbose, Saccharose und/oder Isomaltulose oxidiert wird.

4. Verfahren nach Anspruch 1, wobei die Umsetzung eine Hydrierung ist.

5. Verfahren nach Anspruch 4, wobei .reduzierende Zucker hydriert werden, insbesondere Glucose, Fructose, Xylose, Sorbose, Isomaltose, Isomaltulose, Trehalulose, Maltose und/oder oder Lactose.

6. Verfahren nach Anspruch 1, wobei die Umsetzung eine reduktive Aminierung ist.

7. Verfahren nach Anspruch 6, wobei reduzierende Zucker reduktiv aminiert werden, insbesondere Glucose, Fructose, Xylose, Sorbose, Isomaltose, Isomaltulose, Trehalulose, Maltose und/oder Lactose.

8. Verfahren nach einem der Ansprüche 1 bis 7, wobei das Metall ein Edelmetall ist, z. B. Platin, Palladium, Rhodium und/oder Ruthenium.

9. Verfahren nach einem der Ansprüche 1 bis 7, wobei das Metall ein Nicht-Edelmetall ist, insbesondere Kupfer und/oder Nickel.

10. Verfahren nach Anspruch 8 oder 9, **dadurch gekennzeichnet, dass** als Metallkatalysator ein Monometallkatalysator verwendet wird.

11. Verfahren nach Anspruch 8 oder 10, **dadurch gekennzeichnet, dass** der Edelmetallkatalysator Platin oder eine Platinlegierung umfasst.

12. Verfahren nach einem der Ansprüche 8 bis 11, **dadurch gekennzeichnet, dass** der Metallkatalysator wenigstens zwei Metalle umfasst.

13. Verfahren nach einem der Ansprüche 8 bis 12, **dadurch gekennzeichnet, dass** der Metallkatalysator wenigstens ein Promotormetall aufweist.

14. Verfahren nach einem der Ansprüche 1 bis 13, **dadurch gekennzeichnet, dass** in die wässrige Phase kontinuierlich oder in geeigneten Zeitabständen eine Zugabe des die Nanopartikel stabilisierenden Polymers erfolgt.

15. Verfahren nach einem der Ansprüche 1 bis 14, **dadurch gekennzeichnet, dass** als Metallkatalysator polymerstabilisierte Nanopartikel verwendet werden, die in einer Membrananordnung gehalten werden.

16. verfahren nach einem der Ansprüche 1 bis 14, **dadurch gekennzeichnet, dass** als Metallkatalysator in einem Trägermaterial immobilisierte polymerstabilisierte Nanopartikel verwendet werden.

17. Verfahren nach Anspruch 16, **dadurch gekennzeichnet, dass** die polymerstabilisierten Nanopartikel in einer Gelstruktur immobilisiert werden.

18. Verfahren nach einem der Ansprüche 1 bis 3 und 8 bis 17, wobei die bei der Oxidation erhaltenen Produkte mittels Elektrodialyse kontinuierlich aus dem Reaktionssystem entfernt und gewonnen werden.

## Claims

1. Method for industrial conversion of carbohydrates, alcohols, aldehydes or polyhydroxy compounds in the aqueous phase, **characterised in that** the conversion is carried out catalytically using a metallic catalyst formed from polymer-stabilised nanoparticles.

2. Method according to claim 1, wherein the conversion is an oxidation.

3. Method according to claim 2, wherein glucose, fructose, sorbose, saccharose and/or isomaltulose is oxidised.

4. Method according to claim 1, wherein the conversion is a hydration.

5. Method according to claim 4, wherein reducing sugars are hydrated, especially glucose, fructose, xylose, sorbose, isomaltose, isomaltulose, trehalulose, maltose and/or lactose.

6. Method according to claim 1, wherein the conversion is a reductive amination.

7. Method according to claim 6, wherein reducing sugars are reductively aminated, especially glucose, fructose, xylose, sorbose, isomaltose, isomaltulose, trehalulose, maltose and/or lactose.

8. Method according to any one of claims 1 to 7, wherein the metal is a precious metal, e.g. platinum, palladium, rhodium and/or ruthenium.

9. Method according to any one of claims 1 to 7, wherein the metal is a non-precious metal, especially copper and/or nickel.

10. Method according to claim 8 or 9, **characterised in that** a mono-metallic catalyst is used as the metallic catalyst.

11. Method according to claim 8 or 10, **characterised in that** the precious-metal catalyst comprises platinum or a platinum alloy.

12. Method according to any one of claims 8 to 11, **characterised in that** the metallic catalyst comprises at least two metals.

13. Method according to any one of claims 8 to 12, **characterised in that** the metallic catalyst provides at least one promoter metal.

14. Method according to any one of claims 1 to 13, **characterised in that** the polymer stabilising the nanoparticles is added to the aqueous phase either continuously or at appropriate intervals.

15. Method according to any one of claims 1 to 14, **characterised in that** polymer-stabilised nanoparticles which are held in a membrane arrangement are used as the metallic catalyst.

16. Method according to any one of claims 1 to 14, **characterised in that** polymer-stabilised nanoparticles immobilised in a carrier material are used as the metallic catalyst.

17. Method according to claim 16, **characterised in that** the polymer-stabilised nanoparticles are immobilised in a gel structure.

18. Method according to any one of claims 1 to 3 and 8 to 17, wherein the products obtained through oxidation are continuously removed and recovered from the reaction system by means of electrodialysis.

## Revendications

1. Procédé de transformation industrielle d'hydrates de carbone, d'alcools, d'aldéhydes ou de composés polyhydroxylés en phase aqueuse, **caractérisé en ce que** la transformation est effectuée par voie catalytique en utilisant un catalyseur métallique constitué de nanoparticules stabilisées avec un polymère.

2. Procédé selon la revendication 1, dans lequel la transformation est une oxydation.

3. Procédé selon la revendication 2, dans lequel le glucose, le fructose, le sorbose, le saccharose et/ou l'isomaltulose sont oxydés.

4. Procédé selon la revendication 1, dans lequel la transformation est une hydrogénation.

5. Procédé selon la revendication 4, dans lequel des sucres réducteurs sont hydrogénés, en particulier le glucose, le fructose, le xylose, le sorbose, l'isomaltose, l'isomaltulose, le tréhalulose, le maltose et/ou le lactose.

6. Procédé selon la revendication 1, dans lequel la transformation est une amination réductrice.

7. Procédé selon la revendication 6, dans lequel des sucres réducteurs sont aminés dans des conditions réductrices, en particulier le glucose, le fructose, le xylose, le sorbose, l'isomaltose, l'isomaltulose, le tréhalulose, le maltose et/ou le lactose.

8. Procédé selon l'une quelconque des revendications 1 à 7, dans lequel le métal est un métal noble, par exemple le platine, le palladium, le rhodium et/ou le ruthénium.

9. Procédé selon l'une quelconque des revendications 1 à 7, dans lequel le métal est un métal non noble, en particulier le cuivre et/ou le nickel.

10. Procédé selon la revendication 8 ou 9, **caractérisé en ce qu'**on utilise comme catalyseur métallique un catalyseur monométallique.

11. Procédé selon la revendication 8 ou 10, **caractérisé en ce que** le catalyseur à base de métal noble comprend du platine ou un alliage du platine.

12. Procédé selon l'une quelconque des revendications 8 à 11, **caractérisé en ce que** le catalyseur métallique comprend au moins deux métaux.

13. Procédé selon l'une quelconque des revendications 8 à 12, **caractérisé en ce que** le catalyseur métallique comprend au moins un métal promoteur.

14. Procédé selon l'une quelconque des revendications 1 à 13, **caractérisé en ce que** dans la phase aqueuse a lieu, en continu ou à intervalles appropriés, une addition du polymère stabilisant les nanoparticules.

15. Procédé selon l'une quelconque des revendications 1 à 14, **caractérisé en ce que** sont utilisées comme catalyseur métallique des nanoparticules stabilisées avec un polymère, qui sont contenues dans un dispositif à membrane.

16. Procédé selon l'une quelconque des revendications 1 à 14, **caractérisé en ce qu'**on utilise comme catalyseur métallique des nanoparticules stabilisées avec un polymère immobilisées dans une matière support.

17. Procédé selon la revendication 16, **caractérisé en ce que** les nanoparticules stabilisées avec un polymère sont immobilisées dans une structure gélifiée.

18. Procédé selon l'une quelconque des revendications 1 à 3 et 8 à 17, dans lequel les produits obtenus lors de l'oxydation sont séparés et extraits du système réactionnel en continu par électrodialyse.
